# EUROPEAN PATENT APPLICATION

(11) **EP 1 911 735 A1**
(43) Date of publication of application: **16.04.2008**
(21) Application number: 06255166.8
(22) Date of filing: 06.10.2006
(51) Int. Cl.: C07C 1/22, C07C 1/24, C10G 3/00

(54) **Process for hydrogenation of carboxylic acids and derivatives to hydrocarbons**

(71) Applicant: BP OIL INTERNATIONAL LIMITED, Sunbury-on-Thames, Middlesex TW16 7BP (GB)
(72) Inventor: The designation of the inventor has not yet been filed
(74) Representative: De Kezel, Eric

(57) **Abstract**

A process for hydrogenating a carboxylic acid and/or derivative thereof having a carboxylate group represented by the general formula R¹COO⁻, which process comprises feeding hydrogen and the carboxylic acid and/or derivative thereof to a reactor and maintaining conditions within the reactor such that hydrogen reacts with the carboxylic acid and/or derivative thereof to produce a product stream comprising carbon dioxide, carbon monoxide, methane and hydrocarbons represented by general formulae R¹H and R¹CH₃, characterised in that the molar ratio of R¹H : R¹CH₃ is above a pre-determined value and/or the mole ratio of the sum of carbon dioxide, carbon monoxide and methane to carboxylate groups is above a pre-determined value.

## Description

This invention relates to the field of hydrogenation, more specifically to a process for the hydrogenation of a carboxylic acid and/or derivative thereof to produce one or more hydrocarbons.

It is widely believed that increased concentrations of atmospheric carbon dioxide can lead to climate change through global warming effects. The burning of fossil fuels is thought to be chiefly responsible for such atmospheric increases, and governments are beginning to set targets for regulating or reducing anthropogenic carbon dioxide emissions in an attempt to mitigate and reduce such emissions.

Liquid fuels, such as gasoline, liquefied petroleum gas (LPG), diesel and aviation fuels, are major sources of atmospheric carbon dioxide emissions. In the main, they are derived from fossil fuels such as crude oil, natural gas and coal. Natural gas and coal, for example, can be converted to syngas through processes such as steam reforming or partial oxidation in which the syngas is subsequently converted into liquid hydrocarbon products by Fischer Tropsch synthesis. Crude oil is typically distilled into various fractions based on different boiling points in a refinery, which fractions can either be used as fuels directly, optionally with a suitable polishing treatment such as hydrotreating for sulphur removal, or can be converted into hydrocarbons suitable for use as fuels through processes such as cracking or reforming. On combustion of mineral fuels, they contribute to increasing carbon-dioxide concentrations in the atmosphere.

In order to mitigate against such problems and concerns, it would be desirable to use fuels that make no net contribution to atmospheric carbon dioxide concentrations. One source of so-called CO₂-neutral fuels is biomass. As biomass is ultimately produced from atmospheric carbon dioxide through photosynthesis and related processes, the carbon dioxide released on its combustion is the same as that used in its initial production.

One type of biomass-derived fuel that is now being used for motor vehicles is biodiesel. One type ofbiodiesel comprises a blend of regular fossil fuel-derived diesel and a biological oil (bio-oil), typically a plant oil such as rapeseed, sunflower or corn oil. Optionally the bio-oils, comprising fatty acid triglycerides, can be converted to a corresponding ester, such as the methyl ester, before use. Blends of mineral diesel with bio-oils or their fatty acid esters have a higher oxygen content than conventional mineral-derived diesel fuel, which can reduces the energy content of the fuel and also its oxidative stability. Additionally, regular combustion engines may need modification before they can be used to bum such blended fuels.

As an alternative, instead of being blended with existing diesel compositions, bio-oils can be hydrogenated to yield hydrocarbons that are suitable for use as a diesel fuel, or as additives to diesel fuel, as described for example in US 5,702,722. As the fuel is predominantly paraffinic, then they have similar energy content and oxidative stability to conventional mineral-derived diesel fuel. Additionally, an existing refinery hydrotreater or hydrocracker can be retrofitted to allow a co-feed of biologically derived oils as a co-feed with an existing refinery-derived hydrocarbon stream, such that the product comprises hydrocarbons derived both from refinery sources and biomass sources, as described by Baldauf & Balfanz in VDE Reports No 1126 (1994) pp153-168.

A problem with hydrogenation of bio-oils is that large quantities of hydrogen are required to produce hydrocarbons. It would therefore be desirable to minimise hydrogen consumption to reduce usage of raw materials and improve process efficiency.

According to a first aspect of the present invention, there is provided a process for hydrogenating a carboxylic acid and/or derivative thereof, which carboxylic acid and/or derivative thereof comprises one or more carboxylate groups with general formula R¹COO, which process comprises feeding hydrogen and the carboxylic acid and/or derivative thereof to a reactor and maintaining conditions within the reactor such that hydrogen reacts with the carboxylic acid and/or derivative thereof to produce a product stream comprising one or more hydrocarbons containing the R¹ group, carbon dioxide, carbon monoxide and methane, characterised in that the molar ratio of the sum of carbon dioxide plus carbon monoxide plus methane produced to the carboxylate groups fed to the reactor is above a pre-determined value.

Without being bound by any theory, it is believed that there are a variety of reaction pathways by which a carboxylic acid can be converted to one or more hydrocarbons. To take an example, a deoxygenation pathway for a fatty acid triglyceride is illustrated in equation I, in which oxygen is removed from the triglyceride as carbon dioxide through decarboxylation.

(R-C(O)-O)₃-C₃H₅ + 3 H₂ → 3RH + 3 CO₂ + C₃H₈ I

An alternative pathway involves hydrogenation of the carboxyl group, which oxygen is removed from the molecule as water, according to equation II.

(R-C(O)-O)₃-C₃H₅ + 12 H₂ → 3 RCH₃ + C₃H₈ + 6 H₂O II

Other reactions that are thought to occur in the reactor are the reduction of CO₂ to carbon monoxide and methane, according to reactions III and IV.

CO₂ + H₂ → CO + H₂O III

CO + 3H₂ → CH₄ + H₂O IV

From these reactions it is apparent that the pathway of equation I, which liberates CO₂ compared to equation II, which involved hydrogenation of the carboxylate group, consumes less hydrogen. Thus, by measuring by-products associated with the carbon dioxide produced as a result of equation I, then the consumption of hydrogen can be minimised. Furthermore, by minimising reduction of carbon dioxide to carbon monoxide and methane, further reduction in hydrogen consumption can be achieved.

Thus, hydrogen consumption can be reduced by minimising the formation of carbon monoxide, methane and water compared to carbon dioxide. Maximising the formation of carbon dioxide compared to other by-products that result from hydrogenation of the carboxylic acid and/or derivative thereof has hitherto typically been avoided as the presence of carbon dioxide can exacerbate corrosion effects in process equipment.

In a typical hydrogenation process, carbon dioxide and other gases, for example carbon monoxide, water, unreacted hydrogen and light hydrocarbons such as methane and propane, are removed from the liquid product stream using a flash separator, in which a vapour fraction comprising the carbon dioxide and other light gases are separated from a liquid fraction comprising hydrocarbons produced from the carboxylic acid and/or derivative thereof. Hitherto, it has been considered advantageous to minimise the formation of CO₂ and other by-product gases, as they build up in the hydrogen recycle loop to the reactor requiring a purge to control their concentration, which lowers hydrogen efficiency. The present invention mitigates this effect by improving hydrogen utilisation in the reactor, and minimising hydrogen consuming side reactions, thus offsetting and typically outweighing any loss of hydrogen in this way.

Water produced in the reaction can also be removed with the vapour fraction of the flash separator, although because it forms a separate liquid phase to the hydrocarbon it can also be separated using a decantation vessel.

By measuring the total concentration of carbon dioxide, carbon monoxide and methane in the product stream, for example in the vapour fraction from the flash separator and comparing this with the quantity of carboxylic acid and/or derivative thereof fed to the reactor, then the percentage of the carboxylate groups that have been converted to hydrocarbons according to equation I can be calculated. Techniques such as gas chromatography or optical techniques such as IR or NIR spectroscopy can be used to measure carbon dioxide, monoxide and methane in the product stream. Flow rate measurements can be used to determine the quantity of carboxylic acid and/or derivative thereof fed to the reactor.

Preferably, the mole ratio of the sum of carbon dioxide plus carbon monoxide plus methane in the product stream to the carboxylate groups fed to the reactor, henceforth referred to as the (CO₂ + CO + CH₄):carboxylate mole ratio, is maintained above a value of 0.1:1, for example maintained at a value of 0.3:1 of above, for example in the range of from 0.3:1 to 0.9:1.

In a further embodiment, the mole ratio of carbon dioxide to sum of carbon monoxide plus methane in the product stream, henceforth referred to as the CO₂:(CO + CH₄) mole ratio, is also above a pre-determined value. Preferably, the ratio is maintained such that the hydrogen consumed through a combination of equations I, III and IV is lower than the hydrogen that would be consumed according to the pathway of equation II. Thus, the CO₂:(CO + CH₄) mole ratio is preferably maintained above a value of 0.3:1, for example in the range of from 0.5:1 to 0.9:1.

The vapour fraction from the flash separator is typically recycled to the reactor so that unreacted hydrogen can be re-used. A purge is typically taken from the recycle stream to prevent build up of potentially damaging impurities.

In one embodiment, the vapour fraction is treated to separate carbon dioxide. This is achievable by using an absorbing material, for example a liquid amine such as mono- or diethanolamine, or a solid basic oxide such as an alkali metal or alkaline earth metal modified zeolite or solid oxide. The absorbent can then be regenerated by various means for re-use, such as by thermal or chemical treatment. The remaining, unabsorbed gases, including the unreacted hydrogen, can then be recycled back to the reactor. Removal of carbon dioxide in this way is advantageous as it allows a smaller purge to be taken from the recycle stream, which helps to improve the hydrogen efficiency of the process.

In another embodiment of the invention, the vapour fraction is contacted with a selectively permeable membrane, that allows the selective separation of hydrogen from the other components of the flash separator. This can eliminate the need for a purge stream, which further improves the hydrogen efficiency of the process.

Carbon dioxide removed from the vapour fraction, either in a purge, through selective absorption or otherwise can optionally be captured for sequestration.

Variables that influence the (CO₂ + CO + CH₄):carboxylate mole ratio include the hydrogen partial pressure in the reactor, the reaction temperature, and the molar ratio of carboxylate groups to hydrogen in the reactor. For example, higher reaction temperatures, higher carboxylate to hydrogen mole ratios, and lower hydrogen partial pressures tend to favour an increased (CO₂ + CO + CH₄):carboxylate mole ratio. Additionally, although CO₂:(CO + CH₄) mole ratios also tend to increase with lower hydrogen partial pressures, hydrogenation tends to increase with increased reaction temperature. Therefore, a balance in reaction conditions needs to be maintained such that the reaction pathway of equation I is favoured over equation II, while mitigating further CO₂ reduction reactions according to equations III and IV.

According to a second aspect of the present invention, there a provided a process for hydrogenating a carboxylic acid and/or derivative thereof having a carboxylate group represented by the general formula R¹COO, which process comprises feeding hydrogen and the carboxylic acid and/or derivative thereof to a reactor and maintaining conditions within the reactor such that hydrogen reacts with the carboxylic acid and/or derivative thereof to produce a product stream comprising hydrocarbons represented by general formulae R¹H and R¹CH₃ characterised in that the molar ratio of R¹H : R¹CH₃ is above a pre-determined value.

The carboxylic acid and/or derivative thereof comprises one or more carboxylate groups represented by the general formula R¹COO. For a carboxylic acid, the formula of the compound can be represented by R¹COOH, for an ester, such as a methyl ester, the formula is R¹COOCH₃, and for a fatty acid triglyceride, the formula can be represented by (R¹COO)₃C₃H₅. On hydrogenation, two different hydrocarbon products derived from the carboxylate group are possible; according to equation I above, one of the hydrocarbons has formula R¹H, such that the carboxylate group liberates CO₂, while the hydrocarbon produced from the pathway described in equation II is R¹CH₃, i.e. with one additional carbon atom compared to that of equation I resulting from hydrogenation of the carboxylate group. By minimising the formation of the larger (R¹CH₃) hydrocarbon, then hydrogen consumption is minimised.

In one embodiment of this second aspect of the present invention, the ratio of R¹H to R¹CH₃ from the product stream or in the liquid fraction of the flash separator can be measured by chromatographic techniques, for example GC or HPLC.

The R¹H : R¹CH₃ mole ratio value is suitably maintained at a value of greater than 0.1:1, for example in the range of 0.1:1 to 3:1. In one embodiment if the invention, the ratio is maintained in the range of from 1:1 to 2:1.

By converting the carboxylic acid and/or derivative thereof into hydrocarbons typically present in existing fuel compositions, then the hydrocarbons so-produced can be used directly as a fuel, or alternatively can be blended or otherwise incorporated into an existing mineral fuel, for example diesel, gasoline or aviation fuel, depending on the boiling range and/or number of carbon atoms in the hydrocarbons. This avoids the need to modify engines or other combustion equipment that would otherwise be required if unreacted carboxylic acid and/or derivative thereof were to be used. Lighter hydrocarbons such as methane and propane that also typically result from the process of the present invention can also be incorporated into a fuel product, for example propane produced on hydrogenation of a triglyceride can be blended with propane produced in a crude oil refinery or from compression of natural gas. Alternatively, any by-products that cannot be captured or separated in sufficient purity can be combusted in order to obtain power there from. This is conveniently achieved by feeding it as fuel to an on-site power station or combined heat and power generator, for example.

The carboxylic acid and/or derivative thereof is an organic compound comprising at least one carboxylate (COO⁻) group. In a carboxylic acid, the carboxylate unit is protonated (COOH). A derivative of the carboxylic acid is a compound that can liberate the corresponding carboxylic acid when hydrolysed, for example an ester or an anhydride. Included in this definition are compounds comprising more than one carboxylate group, for example di-carboxylic acids, di-esters, or di- or tri-glycerides.

The carboxylic acid and/or derivative thereof is preferably chosen such that the boiling point characteristics and/or the number of carbon atoms in the hydrocarbons resulting from their hydrogenation are in the same range as those of the target fuel product. For example, diesel fuels typically comprise hydrocarbons with in the range of from 10 to 25 carbon atoms. In a preferred embodiment of the invention, fatty acids and/or their esters are used. Such compounds have general formula R¹C(O)OH and/or R¹C(O)O-R², where R¹ is typically a long hydrocarbon chain. Examples of fatty acids and/or esters suitable for producing hydrocarbons suitable for use as diesel fuel include, lauric, myristic, palmitic, stearic, linoleic, linolenic, oleic, arachidic and erucic acids and/or esters thereof, wherein R¹ comprises 11, 13, 15, 17, 17, 17, 17, 19 and 21 carbon atoms respectively. The esters may comprise R² groups with in the range of from 1 to 6 carbon atoms, for example methyl, ethyl, propyl or butyl, or alternatively the ester may be a mono-, di- or triglyceride, with general formula [R¹C(O)O]ₙC₃H₅(OH)₃₋ₙ, where n = 1, 2 or 3 for mono-, di- or triglycerides respectively. The fatty acids and/or esters thereof may have saturated or unsaturated hydrocarbon groups. Di- or tri-glycerides may comprise hydrocarbon chains derived from the same or different fatty acids. A mixture of more than one carboxylic acid and/or derivative thereof can be fed to the reactor.

In one embodiment of the invention, the carboxylic acid and/or derivative thereof is derived from biomass, being a component for example of plant or animal-derived oil or fat. Use of biologically-derived carboxylic acids and/or esters ensures that the resulting fuel composition has a lower net emission of atmospheric carbon dioxide compared to an equivalent fuel derived purely from mineral sources. Suitable biological sources of fatty acids and/or esters include plant-derived oils, such as rapeseed oil, peanut oil, canola oil, sunflower oil, tall oil, corn oil, soybean oil and olive oil. Animal oils or fats, such as fish oil, lard, tallow, chicken fat, or milk and milk-derived products, are also suitable sources of fatty acids and/or esters, as are oils derived from microorganisms, for example microalgae. Waste oils, such as used cooking oils can also be used.

The process of the present invention can be used for the co-hydrogenation of a carboxylic acid and/or derivative thereof with a hydrocarbon-containing stream. Hydrocarbon-containing streams, such as those produced in a crude oil refinery, are typically hydrogenated by processes such as hydrotreating or hydrocracking to remove heteroatom-containing compounds, such as nitrogen or sulphur-containing compounds, which can otherwise cause harmful emissions when a fuel is combusted. An advantage of the process therefore is that it can be readily retrofitted to an existing hydrotreating or hydrocracking reactor.

Hydrotreating or hydrocracking of a refinery-derived hydrocarbon stream is typically carried out at temperatures in the range of from 200 to 430°C and pressures of up to 200 bara (20 MPa), typically above 1 bara (0.1 MPa). Such conditions are suitable for hydrogenating a carboxylic acid and/or derivative thereof in accordance with the present invention, whether or not it is hydrogenated in combination with a refinery hydrocarbon stream. In a preferred embodiment, reaction temperatures in the range of from 200 to 410°C are maintained, preferably in the range of from 320°C to 410°C. Reaction pressures below 100 bara (10 MPa), such as 50 bara (5 MPa) or below, are preferably used. In one embodiment, the reaction pressure is 30 bara (3 MPa) or below. Hydrotreating conditions are preferred as less cracking of the one or more hydrocarbons derived from the carboxylic acid and or ester thereof tends to occur. Additionally, hydrotreating conditions typically result in lower consumption of hydrogen and higher CO₂ to carboxylate mole ratios.

The hydrogen partial pressure is preferably maintained at a value of less than 100 bara (10 MPa), for example 50 bara (5 MPa) or below. In one embodiment of the invention, the partial pressure is maintained at a value of 30 bara (3MPa) or less. The hydrogen can be introduced into the reactor as a fresh feed, or can be recycled from other parts of the process. Preferably the hydrogen is substantially pure, although some contamination in a recycled hydrogen source is possible, for example with by-products recycled from the vapour fraction of the flash separator.

Conditions are preferably maintained in the reactor such that almost complete conversion of the carboxylic acid and/or derivative thereof is achieved, for example greater than 90wt% conversion, preferably greater than 95% conversion. In embodiments of the invention involving co-hydrogenation of the carboxylic acid and/or reactive derivative thereof with a refinery-derived hydrocarbon stream, the severity of the conditions may also depend on the nature of the hydrocarbon-containing process stream being fed to the reactor, and also the nature of the desired fuel product.

The hydrogenation reaction may be catalysed or uncatalysed, preferably catalysed. Suitable catalysts include those comprising one or more of Pd, Pt, Ni, Ru, Cu, Cr, Fe, Co, Mo and W, for example catalysts comprising Ni or Co in combination with Mo. A CoMo catalyst is preferred over a NiMo catalyst, as its activity tends to be lower, which favours the lower hydrogen-consuming reaction of equation I, and also reduces the tendency for hydrogen consuming side-reactions such as those represented by equations III and IV. The catalyst is typically supported on a support such as silica, zirconia, titania or gamma-alumina, preferably gamma-alumina. Such catalysts are suitable under both hydrotreating and hydrocracking, conditions.

The liquid fraction from the flash separator is typically fed to a further separation unit, for example a fractionation or distillation unit, to separate the hydrocarbon mixture into various fuel fractions comprising gasoline, diesel or kerosene, for example.

A low partial pressure of hydrogen can be used in order to maintain the carbon dioxide to carboxylate group mole ratio to a value at or above the pre-determined value. However, often low hydrogen partial pressures can lead to deactivation of hydrogenation catalysts. Therefore, in one embodiment of the invention employing a fixed bed catalyst, hydrogen is separately and simultaneously injected at two or more different depths of the catalyst bed. This provides periodic increases in the hydrogen partial pressure at lower portions of the catalyst bed, which may otherwise be exposed to potentially damaging low concentrations of hydrogen resulting from its consumption higher up in the catalyst bed. In an alternative embodiment, a series of reactors arranged in series is provided, with a means of separating gases from the product stream between the reactors. Fresh hydrogen and/or recovered and purified recycled hydrogen is fed to each of the reactors, minimising the quantity of catalyst exposed to low and potentially damaging partial pressures of hydrogen. In this embodiment, the quantity of catalyst in each of the fixed catalyst beds is typically lower than embodiments comprising a single fixed bed reactor. The gases removed from the liquid portion of the product stream between the reactors can be treated to recover hydrogen for recycling, and to remove carbon dioxide for sequestration. In this embodiment, the pre-determined ratio of carbon dioxide to carboxylate is calculated from the sum of carbon dioxide produced in all the reactors.

In yet another embodiment of the invention, the catalyst is fluidised in the reaction medium, wherein the catalyst is removed, either continuously or batchwise, and regenerated (for example in a stream of carbon dioxide-free hydrogen) and fed back to the reactor in order to maintain catalyst activity.

The process of the present invention may comprise co-hydrotreatment of a biological oil or fat with a fuel precursor hydrocarbon stream derived from a crude oil refinery, which typically comprises middle distillate hydrocarbons which boil at temperatures typically in the range of from 150 to 400°C, and having carbon atom numbers typically in the range of from 10 to 22 carbon atoms. Typically, the fuel precursor hydrocarbon stream is a straight-run fraction from a crude distillation unit, although it may optionally alternatively or additionally comprise hydrocarbons produced by other refinery processes, such as steam cracking and/or hydrocracking of heavier crude fractions, which produce hydrocarbons in a similar boiling range to that of the straight-run fraction. The fuel hydrocarbon precursor stream is preferably derived from a straight run crude oil refinery stream that is suitable for producing diesel fuel and/or kerosene, preferably diesel fuel.

The hydrogenated product, after any flash separation and optional fractionation stages, predominantly comprises one or more hydrocarbons suitable for use as and/or suitable for blending with a diesel fuel. In one embodiment, the percentage by weight of the biological oil or fat fed to the reactor is typically up to 50% of the total liquid feed to the reactor, for example in the range of from 0.1 to 50% by weight or in the range of from 1 to 35% by weight. The biological oil or fat can be fed to the reactor separately from the diesel precursor hydrocarbon-containing stream, or alternatively it can be fed together with all or a portion of the diesel precursor hydrocarbon stream. The latter case is suitable where the biological oil or fat is highly viscous, even after moderate heating, for example in the case of tallow or lard.

A typical fuel hydrocarbon precursor stream derived from a crude oil refinery comprises alkanes, olefins and/or one or more heteroatom-containing compounds. The heteroatom-containing compounds include sulphur-containing compounds such as sulphides, thiophenes, benzothiophenes and mercaptans. They are typically present at concentrations of 200ppm or more, such as 0.1 % by weight or more, for example in the range of from 0.2 to 2% by weight, expressed as elemental sulphur. Olefins may be present in the fuel hydrocarbon precursor stream, for example at concentrations of 0.01 % or more, and may be up to 20% by weight, for example up to 10% by weight or up to 5% by weight. Other possible constituents of the first hydrogen-containing product stream include aromatic compounds, such as naphthenes.

Sulphur concentrations remaining in the hydrocarbon-containing liquid fraction of the flash separator are typically 200ppm or less, expressed as elemental sulphur, for example in the range from from 0.1 to 200ppm. Furthermore, olefins concentrations are typically lower than 1wt%, for example 0.1 wt% or less. The liquid fraction comprises hydrocarbons resulting from the hydrotreated fuel hydrocarbon precursor stream, and also one or more hydrocarbons resulting from the carboxylic acid and/or derivative thereof. This is optionally and preferably fractionated to provide fuel hydrocarbons of different boiling ranges, preferably the predominant portion being in the diesel boiling range.

The invention is now illustrated by the following non-limiting examples, and with reference to the Figures in which;
Figure 1 schematically illustrates a hydrogenation in accordance with the present invention, in which the vapour fraction from the flash separator is recycled, with a purge, to the reactor without carbon dioxide separation;
Figure 2 schematically illustrates a different hydrogenation process in accordance with the present invention, in which carbon dioxide is separated from the recycled vapour fraction before being returned to the reactor;
Figure 3 schematically illustrates a further hydrogenation process in accordance with the present invention in which hydrogen is injected into the reactor at different depths of a fixed catalyst bed; and
Figure 4 shows schematically the apparatus used in the experimental examples.

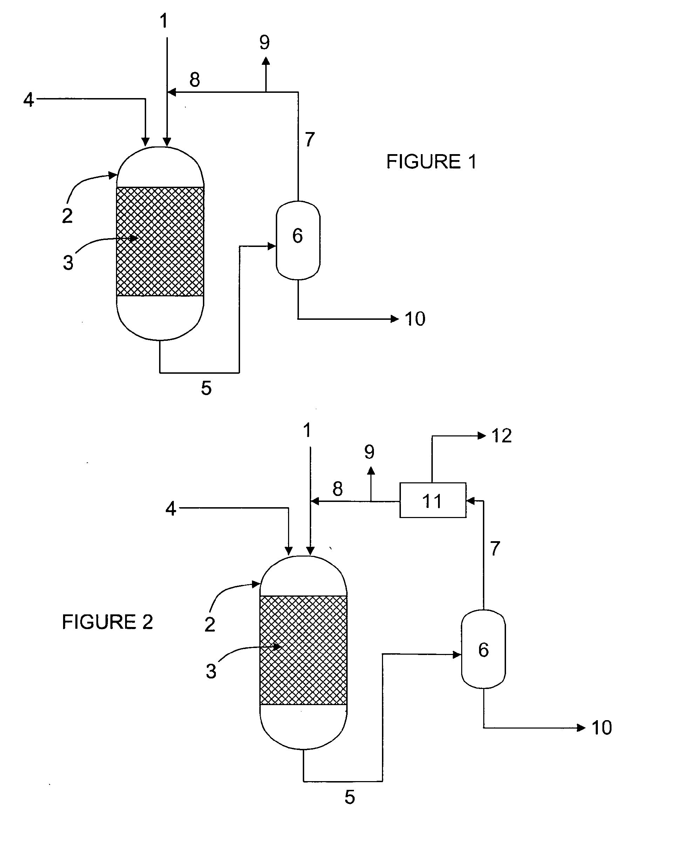

In Figure 1, freshly introduced hydrogen 1 is fed to reactor 2 in addition to a liquid feed of biological oil or fat. The reactor 2 comprises a fixed catalyst bed 3, which is typically a gamma-alumina supported nickel-molybdenum or cobalt-molybdenum hydrogenation catalyst. A biological oil or fat is introduced in liquid form to the reactor through line 4. A product stream 5 is removed from the reactor and fed to a flash separator 6, operating at a lower pressure compared to the reactor. A vapour fraction 7 comprising carbon dioxide, carbon monoxide, methane, propane, water and unreacted hydrogen is removed from the flash separator and recycled through line 8 to the reactor. A purge stream 9 is taken from the recycle line to prevent build up of unwanted byproducts in the recycled hydrogen. A liquid fraction 10 comprising hydrocarbons derived from the biological oil or fat is removed from the flash separator.

In Figure 2, a carbon dioxide separator 11 is inserted between the purge line 9 and the flash separator 6. The carbon dioxide separator contains an amine which absorbs carbon dioxide, and also some of the water from the vapour fraction of the flash separator 7. The amine is transferred through line 12 to be regenerated and reused. The carbon dioxide separated there from is stored for sequestration. The remainder of the vapour fraction is fed back to the reactor through line 8, with a purge 9 to prevent accumulation of unwanted by-products such as carbon monoxide, methane and propane in the recycled hydrogen.

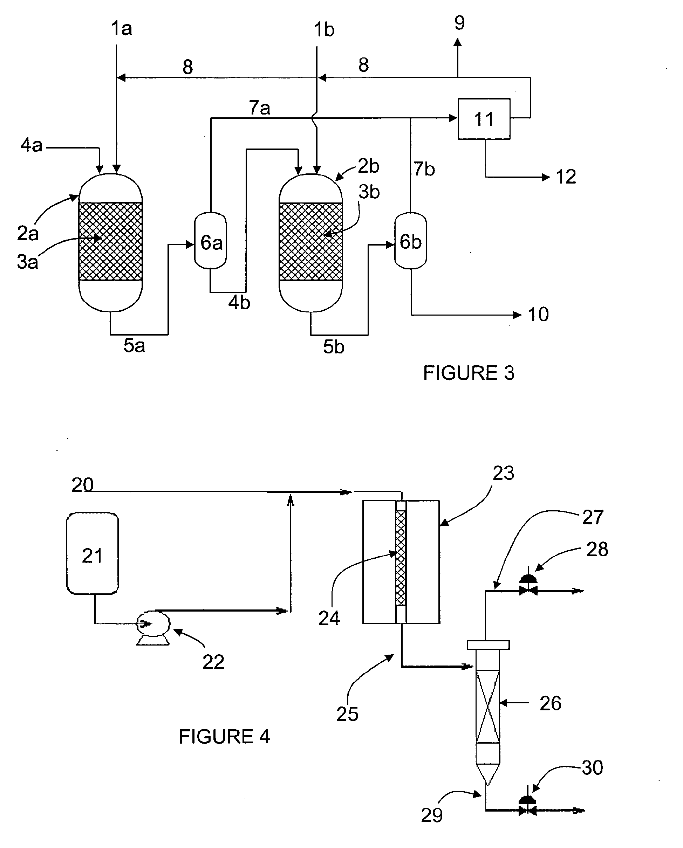

Figure 3 illustrates a process in which hydrogen is fed to a first reactor 2a, containing a first fixed bed of hydrogenation catalyst 3a, through line 1a. Biological oil or fat is also fed to the reactor 3a though line 4a. The first product stream 5a is passed on to a first flash separator 6a. A first vapour fraction is removed through line 7a and passed to carbon dioxide separator 11. A first liquid fraction, comprising hydrocarbons and unreacted biological oil or fat, is passed on through line 4b to second reactor 2b with a second fixed bed hydrogenation catalyst 3b. A second product stream, richer in hydrocarbons derived from biological oil or fat than the first product stream is fed to second flash separator 6b, where a vapour fraction is removed through line 7b and passed on to carbon dioxide separator 11. A second liquid fraction 10 comprising the hydrocarbons is removed from the flash separator. Carbon dioxide and at least some water is removed from the combined first and second vapour fractions by the carbon dioxide separator using an amine. The amine is removed from the separator through line 12 for regeneration and carbon dioxide retrieval and storage. The remaining unabsorbed hydrogen-containing vapour fraction is removed through line 8 and recycled to the first and second reactors via line 8, with a purge 9 to prevent build up of unwanted impurities.

Figure 4 shows schematically the apparatus used for performing the experiments described below. Hydrogen is fed through line 20 to reactor 23. A Liquid oil feed, comprising fuel and/or bio-oil is fed from liquid store 21 via pump 22 into the reactor feed line 20. The reactor has a volume of 114mL and an internal diameter of 14.7mm, and is loaded with cobalt-alumina catalyst 24. Reactor products are fed via line 25 to a flash separator 26, from which a vapour fraction is extracted through line 27 and a liquid fraction is extracted through line 29. The vapour and liquid fraction flow rates are controlled using pressure control valves 28 and 30, which also control the pressure within the flash separator.

### Experiment 1

A liquid feed mixture of 69.74 wt% decalin (C₁₀H₁₈), 0.26wt% dimethyl disulphide (DMDS) and 30wt% tallow oil was prepared. The tallow oil comprised fatty acid chains with 12 to 20 carbon atoms (including the carboxyl carbon), the bulk of the molecules having 16 or 18 carbon atoms in the fatty acid chain (including the carboxyl carbon).

The liquid mixture was fed to a reactor as illustrated in Figure 4, operating at 363°C and 30 barg (3.1 MPa) pressure, at a feed-rate of 60mL/hour. A cobalt-molybdenum on alumina catalyst was used. The liquid hourly space velocity (LHSV) of the liquid feed over the catalyst was 4 h⁻¹. A flow of hydrogen was also fed to the reactor, such that the ratio of H₂ gas volume to liquid feedstock volume was maintained at a value of 200 Nm³/m³ (gas volume at 15.6°C and 1 atm). Reaction was maintained over a period of 5 days. Liquid samples were collected daily and analysed according to a chromatographic method described in ASTM D2887, and also by GCMS. Gaseous off-gas samples were analysed using gas chromatography. The quantity of liquid product was determined gravimetrically. Off-gas volume was measured using a wet-test flow meter.

The mass balance calculated from the quantities of the identified components of the obtained liquid and gaseous products was 99% with 1% standard deviation. The carbon balance was 100% with 1% standard deviation.

### Experiment 2

The same procedure as Experiment 1 was followed, except that the reactor pressure was maintained at 100 barg (10.1 MPa).

### Experiment 3

The same procedure as Experiment 1 was followed, except that the feed was 99.74% decalin and 0.26% DMDS (i.e. no tallow), and the LHSV was 2 h⁻¹.

**Table 1: Products in the liquid phase for tallow hydrogenation experiments.**

| **Compound** | **Carbon atoms** | **Experiment 1 (30 barg)** | **Experiment 2 (100 barg)** |
|---|---|---|---|
| Heptane | | 0.03 | 0.10 |
| Octane | | 0.04 | 0.02 |
| Cyclohexane, 1,3,5-trimethyl- | | | 0.02 |
| Nonane | | 0.10 | 0.03 |
| Decane | | 0.13 | |
| Naphthalene, decahydro-, trans- (decalin) | | 45.75 | 46.32 |
| 1,1'-Bicyclopentyl | | | 0.08 |
| Naphthalene, decahydro-, cis- (decalin) | | 26.82 | 27.49 |
| 2-Methyldecalin (probably trans) | | | 0.02 |
| Unknown | | 0.12 | 0.03 |
| Naphthalene, decahydro-2-methyl- | | | 0.01 |
| Naphthalene, 1,2,3,4-tetrahydro-(tetralin) | | 1.37 | 0.45 |
| Unknown | | | 0.02 |
| Naphthalene | | 0.45 | 0.02 |
| Unknown | | 0.04 | 0.03 |
| Dodecane | 12 | 0.08 | 0.05 |
| Dodecane, 2-methyl- | 13 | 0.04 | 0.02 |
| Tridecane | 13 | 0.70 | 0.43 |
| Tridecane, 2-methyl- | 14 | 0.10 | 0.07 |
| Tridecane, 3-methyl- | 14 | 0.08 | 0.05 |
| Tetradecane | 14 | 0.59 | 0.51 |
| Tetradecane, 2-methyl- | 15 | 0.13 | 0.11 |
| Tetradecane, 3-methyl- | 15 | 0.05 | 0.05 |
| Pentadecane | 15 | 3.95 | 2.92 |
| Dodecane, 2-methyl-8-propyl- | 16 | 0.12 | |
| Pentadecane, 2-methyl- | 16 | | 0.10 |
| Pentadecane, 3-methyl- | 16 | 0.11 | 0.08 |
| Hexadecane | 16 | 2.83 | 3.57 |
| unknown | | 0.05 | 0.03 |
| unknown | | | 0.03 |
| Hexadecane, 2-methyl- | 17 | 0.07 | 0.08 |
| Hexadecane, 3-methyl- | 17 | 0.09 | 0.09 |
| Heptadecane | 17 | 9.50 | 7.90 |
| Pentadecane, 2,6,10-trimethyl- | 18 | 0.08 | |
| Heptadecane, 2-methyl- | 18 | | 0.06 |
| Heptadecane, 3-methyl- | 18 | | 0.03 |
| Cyclohexane, undecyl- | 17 | | 0.03 |
| 1-Octadecene | 18 | | 0.03 |
| Docosane | 22 | | 0.03 |
| Octadecane | 18 | 6.01 | 8.80 |
| Hexadecane, 2,6-dimethyl- | 18 | 0.04 | |
| Hexadecane, 2,6,10,14-tetramethyl- | 20 | | 0.06 |
| Nonadecane | 19 | 0.15 | 0.14 |
| Eicosane | 20 | 0.08 | 0.08 |
| Eicosane, 2-methyl- | 20 | 0.04 | 0.03 |
| Docosane | 22 | 0.04 | 0.02 |
| Tricosane | 23 | 0.04 | 0.01 |
| Tetracosane | 24 | 0.05 | |
| Pentacosane | 25 | 0.06 | |
| Hexacosane | 26 | 0.10 | |
| TOTAL | | 100.00 | 100.00 |

The resulting compositions of the liquid product streams at the end of day 5 for each of experiments 1 and 2 are shown in table 1. From experiment 3, decalin was shown to produce tetralin and naphthalene, but no other hydrocarbons.

Liquid hydrocarbon yield was between 94 and 95% based on the total liquid feed. Tallow-derived products constituted 26% by weight of the total products, which represents a liquid product yield from tallow of approximately 81%. No significant amounts of unconverted tallow oil were present in the product.

Table 2 shows the distribution of carbon numbers of fatty acid groups present in a typical sample of tallow oil. Only even-numbered chains (which includes the carboxyl group) are present.

**Table 2: Typical distribution of fatty acid groups in tallow oil.**

| | |
|---|---|
| Number of Carbon atoms in Fatty Acid Groups* | Percentage in Tallow Oil (wt%) |
| C12 | 0.5 |
| C14 | 3.6 |
| C16 | 27.7 |
| C18 | 62.5 |
| C20 | 0.4 |
| C22 | 0.04 |

| | |
|---|---|
| * Including carboxyl group. | |

Table 3 shows the distribution of hydrocarbons produced from the tallow, demonstrating that the distribution of tallow-derived hydrocarbons in the product are consistent with distribution of the fatty acid chains in the tallow oil.

**Table 3: Distribution of hydrocarbons derived from tallow.**

| Hydrocarbon Ratio | Experiment 1 30 Barg | Experiment 2 100 Bar |
|---|---|---|
| (C13+C14)/(C13 to C18) | 5% | 4% |
| (C15+C16)/(C13 to C18) | 29% | 27% |
| (C17+C18)/(C13 to C 18) | 66% | 69% |

Table 4 shows the extent of decarboxylation (c.f. equation 1) compared to carboxyl hydrogenation (c.f. equation 2) under the two different sets of conditions.

**Table 4: Ratio of tallow-derived hydrocarbons produced by decarboxylation compared to carboxyl hydrogenation.**

| Degree of Decarboxylation | Experiment 1 (30 barg) | Experiment 2 (100 barg) |
|---|---|---|
| C13/(C13+C14) | 54% | 46% |
| C15/(C15+C16) | 58% | 45% |
| C17/(C17+C18) | 61% | 47% |

The results show that the pathway of equation 1, which consumes less hydrogen, is favoured at lower reaction pressures, and lower hydrogen partial pressures, and can be determined from the ratio of hydrocarbons having odd and even numbers of carbon atoms.

Table 5 shows the analysis of the gaseous products from examples 1, 2 and 3. Carbon dioxide yield is clearly higher at the lower reaction pressure.

**Table 5: Yield of gaseous components.**

| | Yield | | |
|---|---|---|---|
| | Experiment 1 | Experiment 2 | Experiment 3* |
| Pressure BarG | 30 | 100 | 30 |
| | | | |
| Carbon Dioxide (wt% of Tallow) | 1.51 | 0.62 | 0.00 |
| Carbon Monoxide (wt% of Tallow) | 0.49 | 0.16 | 0.00 |
| Methane (wt% of Tallow) | 0.13 | 0.30 | 0.02 |
| Total "C1-Carbon" (wt% of Tallow) | 2.40 | 1.52 | |
| Max theoretical "C1 " wt% on Tallow | 4.18 | 4.18 | |
| mol% of tallow carboxyl groups converted to "C1" compounds | 57 | 37 | |
| | | | |
| Total "C1-Carbon" (wt% of Total Feed) | 0.72 | 0.46 | |
| | | | |
| CO₂/(CO₂+CO+CH₄) mole ratio | 0.57 | 0.37 | |
| CO₂/(CO+CH₄) mole ratio | 1.334 | 0.58 | |

| | | | |
|---|---|---|---|
| *wt% decalin. | | | |

These results show that the lower pressure reaction produces not only more carbon dioxide than the higher pressure reaction, but also the proportion of carbon dioxide to hydrogenated by-products thereof (i.e. carbon monoxide and methane) is also higher under lower pressure reaction conditions. Furthermore, lower pressure operation results in a greater proportion of carboxyl groups being converted to CO₂ and other "C1" compounds.

## Claims

1. A process for hydrogenating a carboxylic acid and/or derivative thereof, which carboxylic acid and/or derivative thereof comprises one or more carboxylate groups of general formula R¹COO, which process comprises feeding hydrogen and the carboxylic acid and/or derivative thereof to a reactor and maintaining conditions within the reactor such that hydrogen reacts with the carboxylic acid and/or derivative thereof to produce a product stream comprising carbon dioxide, carbon monoxide, methane and hydrocarbons represented by general formulae R¹H and R¹CH₃containing the R¹ group, **characterised in that** the molar ratio of the sum of carbon dioxide plus carbon monoxide plus methane produced to the carboxylate groups fed to the reactor is above a first pre-determined value.

2. A process as claimed in claim 1, in which the molar ratio is maintained above the first pre-determined value.

3. A process as claimed in claim 1 or claim 2, in which the first pre-determined value is above 0.1:1.

4. A process as claimed in any one of claims 1 to 3, in which the mole ratio of carbon dioxide to the sum of carbon monoxide and methane is above a second pre-detemined value.

5. A process as claimed in claim 4, in which the second pre-determined value is above 0.3:1.

6. A process for hydrogenating a carboxylic acid and/or derivative thereof having a carboxylate group represented by the general formula R¹COO⁻, which process comprises feeding hydrogen and the carboxylic acid and/or derivative thereof to a reactor and maintaining conditions within the reactor such that hydrogen reacts with the carboxylic acid and/or derivative thereof to produce a product stream comprising carbon dioxide, carbon monoxide, methane and hydrocarbons represented by general formulae R¹H and R¹CH₃, **characterised in that** the molar ratio of R¹H : R¹CH₃ is above a third pre-determined value.

7. A process as claimed in claim 6, in which the molar ratio is maintained above third third pre-determined value.

8. A process as claimed in claim 6 or claim 7, in which the third pre-determined value is above 0.1:1.

9. A process as claimed in any one of claims 1 to 8, in which both the molar ratio of the sum of of carbon dioxide plus carbon monoxide plus methane produced to the carboxylate groups fed to the reactor is maintained above the first pre-determined value, and the molar ratio of R¹H : R¹CH₃ is maintained above the third pre-determined value.

10. A process as claimed in any one of claims 1 to 9, in which the carboxylic acid and/or derivative thereof is a fatty acid and/or ester thereof.

11. A process as claimed in any one of claims 1 to 10, in which the carboxylic acid and/or derivative thereof is a component of a biological oil or fat.

12. A process as claimed in claim 11, in which the biological oil or fat is animal-derived.

13. A process as claimed in any one of claims 1 to 12, in which the number of carbon atoms in the one or more hydrocarbons is in the range of from 10 to 22 carbon atoms.

14. A process as claimed in any one of claims 1 to 13, in which the one or more hydrocarbons are suitable for use as and/or for blending with a diesel fuel.

15. A process as claimed in any one of claims 1 to 14, in which the product stream is fed from the reactor to a flash separator, from which a vapour fraction comprising carbon dioxide and unreacted hydrogen is separated from a liquid fraction comprising one or more hydrocarbons derived from the carboxylic acid and/or derivative thereof.

16. A process as claimed in claim 15, in which the liquid fraction from the flash separator is fed to a fractionation column to recover a hydrocarbon fraction suitable for use as and/or suitable for blending with a diesel fuel.

17. A process as claimed in claim 15 or 16, in which the quantities of carbon dioxide, carbon monoxide and methane are measured in the vapour fraction of the flash separator.

18. A process as claimed in any one of claims 15 to 17, in which the R¹H:R¹CH₃ mole ratio is measured in the liquid fraction of the flash separator.

19. A process as claimed in any one of claims 15 to 18, in which the vapour fraction is recycled to the reactor, with the removal of a purge stream.

20. A process as claimed in any one of claims 15 to 19, in which the vapour fraction of the flash separator is treated to remove carbon dioxide therefrom, and the remaining components are recycled back to the reactor.

21. A process as claimed in any one of claims 15 to 19, in which the vapour fraction of the flash separator is contacted with a selective hydrogen-permeable membrane, in which hydrogen is separated and recycled to the reactor.

22. A process as claimed in any one of claims 15 to 21, in which carbon dioxide removed from the vapour fraction is captured for sequestration.

23. A process as claimed in any one of claims 1 to 22, in which the hydrogenation reaction is catalysed.

24. A process as claimed in claim 23, in which the catalyst comprises Mo in combination with Co or Ni.

25. A process as claimed in any one of claims 1 to 24, in which a hydrocarbon stream derived from a crude oil refinery is also fed to the reactor.

26. A process as claimed in claim 25, in which the crude oil refinery-derived hydrocarbon stream is a middle distillate stream.

27. A process as claimed in claim 25 or claim 26, in which the carboxylic acid and/or derivative thereof is a biological oil or fat and comprises up to 50% by weight of the total liquid feed to the reactor.

28. A process as claimed in any one of claims 25 to 27, in which the catalyst is a fixed bed catalyst and hydrogen is introduced at two or more different depths of catalyst bed.

29. A process as claimed in any one of claims 25 to 27, in which there is a plurality of reactors arranged in series, each reactor having a fixed bed catalyst, wherein a supply of hydrogen is fed separately to each reactor, the carboxylic acid and/or reactive derivative thereof is fed to the first reactor, and between each reactor there is a means for separating a vapour fraction comprising unreacted hydrogen and carbon dioxide from a liquid fraction comprising unreacted carboxylic acid and/or derivative thereof, which liquid fraction is fed to the subsequent reactor.

30. A process as claimed in any one claims 1 to 29, in which the reaction temperature is in the range of from 200 to 410°C, and the reaction pressure is up to 200 bara.

31. A process as claimed in claim 30, in which the reaction pressure is 100 bara or less.

32. A process as claimed in claim 31, in which the reaction pressure is 50 bara or less.

33. A process as claimed in claim 32, in which the reaction pressure is 30 bara or less.
